# EUROPEAN PATENT APPLICATION

(11) **EP 2 907 533 A1**
(43) Date of publication of application: **19.08.2015**
(21) Application number: 13844860.0
(22) Date of filing: 03.10.2013
(51) Int. Cl.: A61M 1/14

(54) **PLUG, MEDICAL MODULE AND MEDICAL SYSTEM**

(30) Priority: 09.10.2012 JP 2012223827
(71) Applicant: Toray Industries, Inc., Tokyo, 103-8666 (JP)
(72) Inventor: OKA Yumi, Otsu-shi Shiga 520-8558 (JP); FUJII Kohei, Otsu-shi Shiga 520-8558 (JP); NAKAMATSU Osamu, Okazaki-shi Aichi 444-8522 (JP)
(74) Representative: Prüfer & Partner GbR European Patent Attorneys
(86) International application number: PCT/JP2013/076900
(87) International publication number: WO 2014/057856

(57) **Abstract**

A plug which is detachably attached to a fluid port of a medical module, wherein a lid portion of the plug comprises a part to be penetrated through which a cylindrical tubular connection part of a fluid treatment device is capable of penetrating the lid portion, in a state where the plug is attached to the fluid port, when the connection part and the fluid port are connected so as to allow fluid to pass therethrough. The part to be penetrated has a thickness of 0.2 to 3.0 mm and is formed with a soft synthetic resin having a flexural elastic modulus of 100 to 1500 MPa or with an elastic material having a Shore A hardness of 40 to 90. Additionally, the plug comprises a connection part sealing part which liquid-tightly abuts the connection part in a state where the connection part is inserted inside the plug. Thus, the medical module and the fluid treatment device can be connected hygienically in a simple manner without removing the plug.

## Description

### Technical Field of the Invention

The present invention relates to a plug detachably attached to a fluid port of a medical module which is connected to a cylindrical tubular connection part of a fluid treatment device so as to allow fluid to pass therethrough, and a medical module attached with this plug to the fluid port, and further, a medical system in which this medical module and the fluid treatment device are connected. As the medical module, a medical tool used for an external circulation medical treatment of a body fluid represented by blood or plasma, a filtration filter for broadly treating a fluid for a medical use by passing the fluid therethrough, etc. can be exemplified.

### Background Art of the Invention

As a conventional technology broadly known, there is a fluid treatment performed by connecting a fluid treatment device to a medical module having a fluid port and passing a fluid through the medical module. This is for carrying out a desired treatment by connecting an adequate module, prepared in accordance with a fluid to be treated or a purpose of the treatment, to a fluid treatment device, and in particular, it is employed for a medical use for broadly treating a fluid such as body fluid, blood, chemicals, or water.

As examples of medical modules, a medical tool used for an external circulation medical treatment of a body fluid represented by blood or plasma, for example, such as an artificial kidney, a blood filtration dialyser, a blood filter, a plasma component separator, a plasma separator, a leukocyte remover and a blood component adsorber, and a module for broadly treating a fluid for a medical use by passing the fluid therethrough, for example, such as a filtration filter for a dialysis liquid, etc., can be exemplified, and in any of those, a functional material, such as hollow yarns or an adsorbing material, is filled inside a body case and the body case has one or more fluid ports for passage of the fluid.

In particular, an endotoxin retentive filter (ETRF) module is used at a condition being connected to a dialysis device for removing a toxin called as endotoxin which is contained in a dialysis liquid, has a plurality of hollow cylindrical fluid ports on a cylindrical vessel, and is filled with a functional material such as hollow yarns or an adsorbing material inside the vessel.

In the removal of endotoxin, the dialysis liquid passes through the inside of the ETRF module and is sent to the dialysis device through the fluid ports. For anticontamination, it is necessary that the connection parts of the dialysis device and the fluid ports are liquid-tightly connected without causing leakage.

Further, because of medical use, it is required to use hygienically, and when the ETRF module is connected to the dialysis device, it is used most carefully so as not to touch the fluid port or the connection part with a hand or so as not to allow unnecessary bacteria to propagate by a long-time exposure of the fluid port in air.

Further, in case where a liquid is filled in the ETRF module in advance, there is a case where a plug is detachably attached to the fluid port so that the liquid does not leak from the fluid port, and in such a case, the connection operation to the dialysis device is performed by removing the plug carefully so as not to touch the fluid port and further so as not to pollute circumstances by the leakage of the filled liquid. Where, as the connection part of the dialysis device, for example, as shown in Non-patent document 1, one configured from a synthetic resin or a metal, in order to connect hygienically and securely, formed from a hard plastic or a stainless, is suitably used.

Although the ETRF module is exchanged after being used in a certain period of time, it becomes a burden for the exchange operation that taking the above-described care is required for each exchange.

Accordingly, as described in Patent documents 1 and 2, a method for connecting a medical module by a simple operation without touching the fluid port with hand. In the method, however, because it is necessary to connect the fluid port at a state being opened, in case where a liquid is filled in the module in advance, not only leakage of the liquid from the fluid port more or less cannot be prevented but also there is a fear that an expected pollution is caused by the exposed state of the fluid port.

Moreover, in order to employ such a connection method, required is an ETRF module having a fluid port formed in a special shape coinciding with a connection part of a holder unit, a broadly used general connection part cannot be attached thereto, and it is poor in compatibility.

Further, Patent document 3 describes that, with respect to the connection method between the fluid port and the connection part, the connection part is penetrated through an inner cap in a plug and is inserted inside it. However, it is unchanged that, when the connection part is connected, it is required to remove a protection cap and to expose the inner cap. Further, a connection part sealing part for preventing liquid leakage from the connection part (for example, an O-ring (14a)) is provided outside the inner cap.

Thus, in case where the operation for removing the plug is required and the connection part sealing part is present outside the lid portion of the plug, the connection part sealing part is always in a state exposed outside, and there is a fear that the module is touched by mistake when holding the module with hand or that propagation of unnecessary bacteria is caused by contact with air.

Further, Patent document 4 describes a plug configured as shown in Fig. 1 and attached to a fluid port 500. In this plug, the thickness of a part in a lid portion 700, which is opened by being pressed with a connection part 600, is great, and described is a structure for sealing the connection part 600 by utilizing the thickness of such a lid portion 700. In this case, however, in order to penetrate the lid portion 700 great in thickness, it is necessary to set the diameter of the connection part 600 small, and it becomes a factor for obstructing the flow of a treatment fluid passing through the inside of the connection part.

### Prior art documents

### Patent documents

Patent document 1: JP-A-2003-320025
Patent document 2: JP-A-11-70164
Patent document 3: JP-B-4,196,994
Patent document 4: JP-A-2005-102782

### Non-patent documents

Non-patent document 1: Product catalog "Coupler" supplied by Nitto Kohki Co., Ltd., (2006, autumn version), p.98

### Summary of the Invention

### Problems to be solved by the Invention

Accordingly, an object of the present invention is to provide a plug being attached to a medical module, which can be connected hygienically without taking a particular care when it is connected to a fluid treatment device, which, even in case where a liquid is filled in the medical module, can be connected securely without causing leakage of the filled liquid, which can securely seal a liquid to be treated even when the liquid is passed at a predetermined amount, and which enables to connect the medical module to a general fluid treatment device even by a conventional method for removing the plug, and a medical module attached with such a plug, and further, a medical system.

### Means for solving the Problems

To achieve the above-described object, a plug according to the present invention has a body portion, a fluid port sealing portion formed in a hollow cylindrical shape which liquid-tightly abuts a fluid port of a medical module, an opening portion, and a lid portion provided at a side opposite to a side of the opening portion for closing and protecting the fluid port, which is detachably attached to the fluid port of the medical module, and is characterized in that
the lid portion comprises a part to be penetrated through which a cylindrical tubular connection part of a fluid treatment device is capable of penetrating the lid portion, in a state where the plug is attached to the fluid port, when the connection part and the fluid port are connected so as to allow fluid to pass therethrough,
the part to be penetrated includes at least a center part in a radial direction of the lid portion, has a thickness of 0.2 mm to 3.0 mm, and is formed with a soft synthetic resin having a flexural elastic modulus of 100 to 1500 MPa or with an elastic material having a Shore A hardness of 40 to 90, and,
further, the plug comprises a connection part sealing part which liquid-tightly abuts the connection part in a state where the connection part is inserted inside the plug.

In the present invention, there is an excellent characteristic as compared with a conventional technology, in that the plug having a lid portion for protecting the fluid port can be connected to the connection part of the fluid treatment device to open a flow path at a state being attached to the fluid port as it is. For this, in the lid portion of the plug, there exists the part to be penetrated which is capable of being penetrated by the connection part. By such a structure, the connection part of the fluid treatment device and the fluid port can be connected without removing a protection cap as described in the aforementioned conventional technology. Moreover, in the present invention, the connection part sealing part for keeping a liquid-tightness by being abutted to the connection part inserted inside the plug is provided at a position where the connection part is inserted inside the lid portion, and this connection part sealing part is liquid-tightly abutted to the connection part. The connection part sealing part is provided preferably at an annular style on the inner wall of the plug and the like, because it is necessary to ensure a flow path for liquid passage even in case of being liquid-tightly abutted to the connection part as described above. As aforementioned, although as the conventional technology it is described to enhance the sealing property with the connection part by thickening the lid portion, an applicable diameter of the connection part is limited to a small diameter, and there is a case where it is difficult to select a material and the like for achieving the penetration of the connection part and a secure sealing. In the present invention, by providing such a connection part sealing part inside the lid portion, a liquid-tight sealing becomes possible even in case where a liquid-tight sealing property cannot be kept only by the lid portion, and further, because the sealing part is not exposed outside the lid portion, a hygienic improvement can be expected. Furthermore, since the part to be penetrated in the lid portion includes at least a center part in the radial direction of the lid portion, has a thickness of 0.2 mm to 3.0 mm, and is formed with a soft synthetic resin having a flexural elastic modulus of 100 to 1500 MPa or with an elastic material having a Shore A hardness of 40 to 90, it is possible to easily close the fluid port by this plug, and easy penetration becomes possible when the connection part is pressed to the part to be penetrated.

Further, the present invention can also provide a plug used for a use where, when a fluid port and a connection part of a fluid treatment device are connected, the connection part penetrates a part to be penetrated in a lid portion of the plug to form a flow path. Namely, the present invention also provides a plug having a body portion, a fluid port sealing portion formed in a hollow cylindrical shape which liquid-tightly abuts a fluid port of a medical module, an opening portion, and a lid portion provided at a side opposite to a side of the opening portion for closing and protecting the fluid port, which is detachably attached to the fluid port of the medical module, characterized in that
the plug comprises a connection part sealing part which liquid-tightly abuts a cylindrical tubular connection part of a fluid treatment device in a state where the connection part is inserted inside the plug, and
the plug is used for a use where the connection part penetrates a part to be penetrated in the lid portion to form a flow path, in a state where the plug is attached to the fluid port, when the fluid port and the connection part are connected so as to allow fluid to pass therethrough without removing the plug from the fluid port.

In such a use, the fluid port and the connection part of the fluid treatment device can be connected, in a state where the plug is attached to the fluid port, without removing the plug from the fluid port. However, because the plug is detachably attached to the fluid port, it is also possible to remove the plug from the fluid port and thereafter connect the fluid port to the connection part of the fluid treatment device, and the manner can be selected by judgment of the operator.

Also in the plug in such a use, it is preferred that the above-described part to be penetrated includes at least a center part in a radial direction of the lid portion, has a thickness of 0.2 mm to 3.0 mm, and is formed with a soft synthetic resin having a flexural elastic modulus of 100 to 1500 MPa or with an elastic material having a Shore A hardness of 40 to 90.

In the plug according to the present invention, a structure is preferably employed wherein the above-described body portion is formed with a synthetic resin in a hollow cylindrical shape, and has the fluid port sealing portion on an inner circumferential surface of the body portion, and the fluid port sealing portion is abutted to an outer circumferential surface of the fluid port when the plug is attached to the fluid port.

Further, a structure is also preferably employed wherein the above-described lid portion is molded integrally with a synthetic resin frame, and the frame and the above-described body portion are integrated by ultrasonic welding.

A medical module according to the present invention is detachably attached with a plug to a fluid port of the medical module, the plug having a body portion, a fluid port sealing portion formed in a hollow cylindrical shape which liquid-tightly abuts the fluid port, an opening portion, and a lid portion provided at a side opposite to a side of the opening portion for closing and protecting the fluid port, and is characterized in that
the lid portion comprises a part to be penetrated through which a cylindrical tubular connection part of a fluid treatment device is capable of penetrating the lid portion, in a state where the plug is attached to the fluid port, when the connection part and the fluid port are connected so as to allow fluid to pass therethrough,
the part to be penetrated includes at least a center part in a radial direction of the lid portion, has a thickness of 0.2 mm to 3.0 mm, and is formed with a soft synthetic resin having a flexural elastic modulus of 100 to 1500 MPa or with an elastic material having a Shore A hardness of 40 to 90, and,
further, the plug comprises a connection part sealing part which liquid-tightly abuts the connection part in a state where the connection part is inserted inside the plug.

Further, the present invention also provides a medical module detachably attached with a plug to a fluid port of the medical module, the plug having a body portion, a fluid port sealing portion formed in a hollow cylindrical shape which liquid-tightly abuts the fluid port, an opening portion, and a lid portion provided at a side opposite to a side of the opening portion for closing and protecting the fluid port, characterized in that
the plug comprises a connection part sealing part which liquid-tightly abuts a cylindrical tubular connection part of a fluid treatment device in a state where the connection part is inserted inside the plug, and
the plug is used for a use where the connection part forms a flow path by penetrating a part to be penetrated in the lid portion, in a state where the plug is attached to the fluid port, when the fluid port and the connection part are connected so as to allow fluid to pass therethrough without removing the plug from the fluid port. In this medical module, a structure can be employed wherein the part to be penetrated includes at least a center part in a radial direction of the lid portion, has a thickness of 0.2 mm to 3.0 mm, and is formed with a soft synthetic resin having a flexural elastic modulus of 100 to 1500 MPa or with an elastic material having a Shore A hardness of 40 to 90.

In the above-described medical module, a structure can be employed wherein the medical module is an endotoxin retentive filter module.

Furthermore, a medical system according to the present invention comprises (a) a medical module detachably attached with a plug to a fluid port of the medical module, the plug having a body portion, a fluid port sealing portion formed in a hollow cylindrical shape which liquid-tightly abuts the fluid port, an opening portion, and a lid portion provided at a side opposite to a side of the opening portion for closing and protecting the fluid port, and (b) a fluid treatment device, and is characterized in that
a cylindrical tubular connection part of the fluid treatment device is penetrated through a part to be penetrated including at least a center part in a radial direction of the lid portion, and inserted inside the plug, and
the inserted connection part is liquid-tightly abutted to a connection part sealing part, which is provided inside the plug, to open a flow path.

### Effect according to the Invention

In the present invention, since it is possible to connect the fluid port and the fluid treatment device at a state where a plug itself for protecting the fluid port is not removed, a plug can be provided, which can be connected hygienically in a simple manner without taking a particular care, which, even in case where a predetermined amount of liquid is filled in a medical module, can seal and can be connected quickly and securely without causing leakage of the filled liquid, and which enables to connect the medical module to a general fluid treatment device even by a conventional method for removing the plug, and a medical module attached with such a plug, and further, a medical system, can be provided.

### Brief explanation of the drawings

[Fig. 1] Fig. 1 is a partial sectional view of a fluid port section showing a state where a connection part is connected to a plug according to a conventional technology.
[Fig. 2] Fig. 2 is a side view partially indicating in section of an ETRF module showing an example wherein a plug according to an embodiment of the present invention is attached to the ETRF module.
[Fig. 3] Fig. 3 is a perspective sectional view showing an example of a connection part formed in a lure-lock shape in a dialysis device.
[Fig. 4] Fig. 4 is a perspective sectional view showing an example of a connection part formed in a slip-in shape in a dialysis device.
[Fig. 5] Fig. 5 is a perspective sectional view showing an example of a connection part formed in a coupler shape in a dialysis device.
[Fig. 6] Fig. 6 is a perspective view showing an example of a fluid port having a shape adaptable to a connection part formed in a coupler shape in a dialysis device.
[Fig. 7] Fig. 7 is a sectional view showing an example of a connection method of a connection part in the present invention.
[Fig. 8] Fig. 8 is a perspective sectional view exemplifying a state where a plug according to an embodiment of the present invention is attached to a fluid port of a medical module.
[Fig. 9] Fig. 9 is a perspective sectional view showing a plug and a connection part according to another embodiment of the present invention.
[Fig. 10] Fig. 10 is a perspective sectional view showing a plug and a connection part according to a further embodiment of the present invention.

### Embodiments for carrying out the Invention

Hereinafter, the present invention will be explained in detail together with embodiments.

A plug according to the present invention is one which is detachably attached to a fluid port of a medical module, which closes the fluid port, and which can protect the fluid port. Here, the "protect the fluid port" means to play a role for preventing that the fluid port or the inside thereof is exposed in air, thereby causing that unnecessary bacteria adhere and propagate, a role for preventing that a person carelessly touches by hand at the time of handling, thereby causing that unnecessary bacteria adhere and propagate, or a role for preventing that, in case where a liquid or a gas is filled in the module, such a fluid leaks outside.

Hereinafter, although a case applying the present invention to a plug of an ETRF module will be explained, the preferred embodiments and exemplifications described below are not restricted to this case.

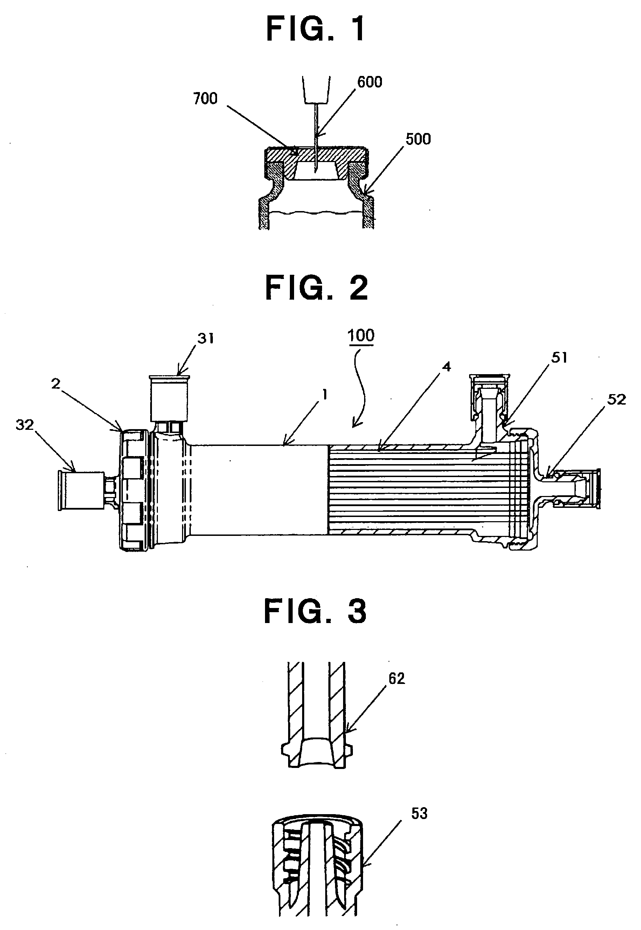

Fig. 2 shows an ETRF module 100 at a state where plugs 31, 32 are attached to fluid ports 51, 52, respectively. ETRF module 100 is a medical module for removing toxins contained in a dialysis liquid, wherein a bundle of hollow yarn membranes 4 are contained in a body case 1 having a cylindrical shape as a functional portion, and the module is filled with water. The formation of the functional portion is not particularly limited to the hollow yarn membranes, and as others, exemplified are adsorption materials, flat membranes, beads, etc.

Fluid ports 51, 52 are provided to ETRF module 100, and although the disposition and the number thereof can be arbitrarily decided in accordance with the usage, generally, as shown in Fig. 2, respective fluid ports are projected respectively at two positions on the ends in the longitudinal direction of body case 1 (for example, on headers 2) and at two positions extending in a direction perpendicular to the axis of the body case 1, and any of the fluid ports communicates with the inside of the module.

Respective plugs are attached to the tips of the respective fluid ports to close and protect the fluid ports. As the material of body case 1, for example, a polyethylene, a polycarbonate, a polystyrene, etc. are suitably used, and by being manufactured by injection molding, it is possible to mold it integrally with the fluid ports and to manufacture it at a high accuracy and at a mass scale.

The connection part of a dialysis device is formed from a synthetic resin or a metal, and in order to connect it hygienically and securely, a hard plastic such as modified polyphenylene ether or a stainless is suitably used therefor. The connection part has a cylindrical shape and is formed as a pipe-like shape having a communication port for liquid passage, and generally a range of 3 mm to 20 mm is suitably employed as the diameter of the section of the communication port.

Although the shape of the fluid port can be arbitrarily set, because the shape of a dialysis liquid side port described in JIS T3250(2012)4.4.4 is widely used in the field of medical fluid treatment, it can be suitably used even in the ETRF module.

Further, by a condition where the connection part has a large communication port as much as possible in a range capable of being inserted into the inner diameter portion of the above-described fluid port at a state where a plug is attached to the fluid port, the flow path resistance at the time of liquid passage can be reduced and a necessary flow rate can be ensured.

For example, since the outer diameter of the dialysis liquid port described in JIS T3250(2012) is 12.4 mm, it is general that the inner diameter thereof is in a range of 8 mm to 10 mm. In case where a plug is attached to such a port, it is preferred that the outer diameter of the portion at which the connection part is about to reach the inside of the port is in a range of 6.0 mm to 9.5 mm, from the viewpoint of securing the flow rate at the time of liquid passage.

Further, it is preferred that the plug has a part to be penetrated through which the above-described plug is capable of penetrating the lid portion of the plug by pressing the plug, in a state where the plug is attached to the above-described fluid port.

Connection parts and fluid ports having various shapes in a dialysis device are exemplified in Figs. 3 to 5.

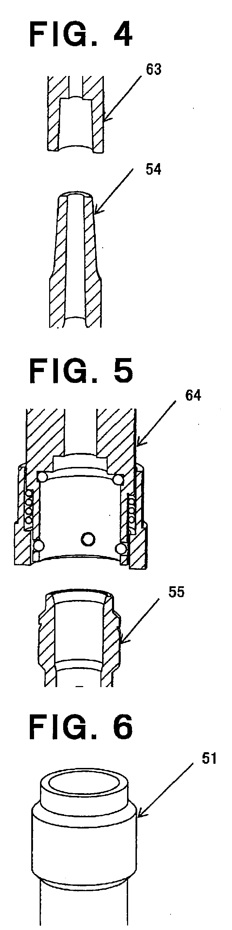

Fig. 3 is a perspective sectional view in case of a lure-lock fluid port 53 and a lure-lock connection part 62, Fig. 4 is a perspective sectional view in case of a slip-in fluid port 54 and a slip-in connection part 63, and Fig. 5 is a perspective sectional view in case of a coupler fluid port 55 and a coupler connection part 64. These are suitably used because of less leakage of liquid, and in particular, the method for using the coupler shape is frequently employed because of a high reliability. For example, in case where the connection part in a dialysis device has a coupler shape, when an example of fluid port 51 of the aforementioned ETRF module 100 having a shape adaptable thereto is shown, it becomes a shape as shown in Fig. 6. Further, in case where the above-described coupler shape is employed, the shape of the fluid port may be set at the above-described shape of the dialysis liquid port described in JIS T3250(2012)4.4.4.

Where, in case where the above-described ETRF module is attached to a dialysis device by a conventional method, an operator first removes the plug by hand, and next, the connection part of the dialysis device is attached to the fluid port of the ETRF module to allow communication. At that time, in case where a liquid is filled in the module, it is necessary to attach the connection part carefully so as not to pollute circumstances by the leakage of the filled liquid. In case where a plurality of fluid ports exist, an operation similar to the above-described operation is required for each of the fluid ports.

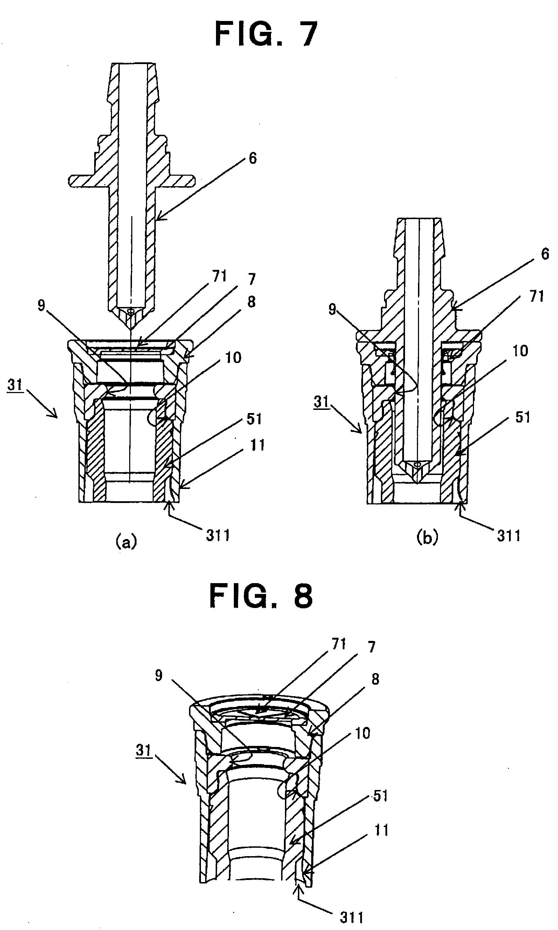

On the other hand, Fig. 7 is a sectional view showing an example of a connection method of a connection part in the present invention, and in the present invention, a structure is employed wherein a connection part 6 penetrates a part to be penetrated 71 in a lid portion 7 to open a flow path, by pressing the connection part 6 to the lid portion 7 provided at a side opposite to the side of an opening portion 311 of plug 31, preferably to a portion near the center thereof, without removing the plug 31 at all, at a state where the plug closes and protects the fluid port 51, and the connection part 6 is inserted inside of the lid portion 7 of the plug 31. Therefore, it can be applied to use in which, when fluid port 51 and connection part 6 of a fluid treatment device are connected, the connection part 6 penetrates part to be penetrated 71 in lid portion 7 to form a flow path.

The outer circumferential portion of connection part 6 inserted inside plug 31 becomes possible to be sealed liquid-tightly by abutting a connection part sealing part 9 present in lid portion 7 of plug 31 over the entire circumference. Similarly, a liquid-tight sealing becomes possible by a condition where a fluid port sealing portion 10 present in plug 31 abuts fluid port 51 over the entire circumference. Where, in Fig. 7, symbol 11 indicates a cover.

In this state, the sealing condition between fluid port 51 and plug 31 is held, and further, connection part 6 is also in a sealing condition with the plug 31.

According to the above-described connection method, since the connection operation can be carried out at a state as it is where plug 31 closes and protects fluid port 51 without removing the plug 31 at all, it becomes possible to allow a liquid to pass through without causing leakage of the filled liquid and without exposing the fluid port 51 outside. In this method, because it is not necessary to remove plugs 31 one by one, a manner can also be employed for connecting fluid port 51 and the dialysis device only by attaching module 100 which is in a state where plug 31 is attached to a holder and the like provided with connection part 6.

Connection part 6 in the above-described connection method has a cylindrical shape having a liquid-passage hole therein, and is configured with a synthetic resin or a metal, and therefor a hard plastic or a stainless is suitably used in order to connect it hygienically and securely. The connection part 6 has a cylindrical shape and is formed in a pipe-like shape having a communication port for liquid passage, and generally one having a diameter of the section of the communication port in a range of 3 mm to 20 mm is suitably employed.

Although connection part 6 in Fig. 7 shows an example of the connection part 6 of a fluid treatment device, which is connected to plug 31 according to the present invention, it is preferred that the tip of the connection part 6 has an angle so as to be able to penetrate lid portion 7 when pressed to the lid portion 7. Although it is preferred that the portion abutting connection part sealing part 9, of the outer circumference or the inner circumference of connection part 6, is flat in the vertical direction in Fig. 7, a step or a rib for abutting plug 31 to regulate the insertion depth of the connection part 6 may be provided. Further, for connection part 6, a cover for protecting the tip thereof may be provided (omitted in the figure).

Fluid port sealing portion 10 may be abutted to any of the outer circumferential surface and the inner circumferential surface of fluid port 51, and in case of being abutted to the outer circumferential surface, if it is formed to be annular, for example, as shown in Fig. 8, it can be tightly contacted by inserting the fluid port 51 into the inner wall thereof. In this case, it is preferred that body portion 8 is formed as a synthetic resin hollow cylindrical body, fluid port sealing portion 10 is surrounded by the synthetic resin body portion 8 by being formed integrally with the inner circumferential wall of the body portion 8, and therefore, the shape thereof is stabilized and the sealing property is enhanced.

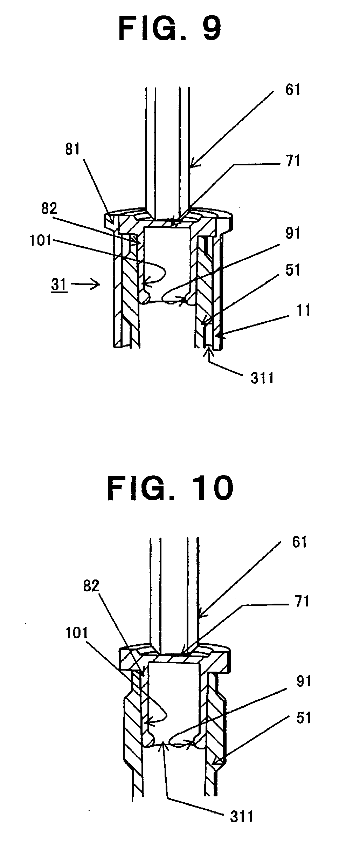

On the other hand, in case of a formation where the fluid port sealing portion abuts the inner circumferential surface of fluid port 51, for example, as shown in Figs. 9 and 10, a fluid port sealing portion 101 is formed in an annular shape in a body portion 82, and the fluid port sealing portion 101 can be tightly contacted by being pressed into the fluid port 51. In the embodiment shown in the figure, when such a fluid port sealing portion 101 is provided, configuration as a single component becomes possible by forming body portion as a synthetic resin single component and forming the fluid port sealing portion 101 so as to project from the lid portion side. In the embodiment shown in the figure, a connection part sealing part 91 is formed at the side of the tip inner circumferential surface of fluid port sealing portion 101.

Further, in the embodiment shown in Fig. 9, a frame 81 made from, for example, a synthetic resin, is provided on the outer circumference at the lid portion side of body portion 82, and cover 11 is molded integrally with the frame 81. In this case, fluid port 51 exists between fluid port sealing portion 101 and cover 11. In the embodiment shown in Fig. 10, a synthetic resin frame as shown in Fig. 9 is not provided, and in this case, for example, a lid portion having part to be penetrated 71, body portion 82, fluid port sealing portion 101 and connection part sealing part 91 can also be formed by an identical material. Although this material is not particularly restricted, for example, a rubber and the like can be used.

The formation as shown in Figs. 7 and 8 where the fluid port sealing portion abuts the outer circumferential surface of the fluid port, as compared with the formation as shown in Figs. 9 and 10 where the fluid port sealing portion abuts the inner circumferential surface of the fluid port, is preferred in a point that the size of a connection device inserted inside is less restricted because the space inside the fluid port sealing portion can be set greater.

Further, in the present invention, it is preferred that the fluid port sealing portion is formed with an elastic material having a Shore A hardness of 40 to 90 because a good sealing property can be obtained. For example, a synthetic rubber such as a silicone rubber, an ethylene-propylene-diene rubber (hereinafter, called as an EPDM rubber) and a nitrile rubber, and an elastomer, can be used. In particular, it is preferred to use a thermoplastic elastomer from the viewpoint capable of manufacturing it at a high accuracy and at a mass scale by using injection molding.

Further, in the present invention, the lid portion is provided at a position for closing the fluid port at a side opposite to the side of the opening portion in the plug. At least a part of the lid portion including the center part thereof forms a part to be penetrated through which the connection part, being pressed at the time of connection with the connection part, can penetrate. Here, the whole of the lid portion may be formed by the part to be penetrated. It is preferred that the part to be penetrated is formed with a soft synthetic resin having a flexural elastic modulus of 100 MPa or more and 1500 MPa or less, preferably 1200 MPa or less, more preferably 1000 MPa or less, or with an elastic material having a Shore A hardness of 40 or more, preferably 70 or more, and 90 or less, preferably 85 or less, and by thus forming, it becomes possible to close the fluid port and to be easily penetrated when the connection part is pressed.

As the material of the lid portion, for example, as the synthetic resin, a resin such as a polypropylene and a polyethylene is preferably used, and as the elastic material, a synthetic rubber such as an EPDM rubber and a nitrile rubber or an elastomer, etc. is preferably used. In particular, it is more preferred to use a thermoplastic elastomer from the viewpoint capable of manufacturing it at a high accuracy and at a mass scale by using injection molding.

Further, it is preferred that the thickness of the part to be penetrated in the lid portion is less than 5.0 mm because of being penetrated with the connection part, more preferably 3.0 mm or less, further preferably 1.5 mm or less, particularly preferably 1.0 mm or less, and by setting at such a thickness, the connection part can easily penetrate. If the thickness of the part to be penetrated is 5.0 mm or more, easiness for opening when the connection part is pressed is damaged. Further, although the lower limit of the thickness is not particularly limited, it is preferred to be 0.1 mm or more in order to keep the strength of the lid portion, and it is more preferred to be 0.2 mm or more.

In case where it is attempted to prevent leakage of a treatment liquid passing through the inside of the connection part utilizing the part to be penetrated at a state where the connection part is penetrated through the plug, it is necessary to increase the contact area between the part to be penetrated and the connection part, and therefore, it is necessarily required to increase the thickness of the part to be penetrated. However, because two requirements with respect to the thickness of the part to be penetrated conflict with each other, it has been difficult to satisfy both of the easiness of opening of the part to be penetrated and the liquid-tightness of the connection part. In the present invention, however, by forming the part to be penetrated as a component with a small thickness and forming a connection part sealing part therein, it has become possible to satisfy both of the easiness of opening of the part to be penetrated and the liquid-tightness of the connection part.

In order to achieve an easy opening when the connection part is pressed, a weak portion becoming a trigger for the opening may be provided in a part of the part to be penetrated in the lid portion, and a weak portion having a thickness of 0.1 mm or more can be provided in a range that does not damage the strength of the lid portion.

Further, in case where the shape of the part to be penetrated in a horizontal direction is a circle or an oval, the inner diameter thereof (in case of an oval, converted into the inner diameter of a hypothetical true circle having the same area) is preferably 7.0 mm or more, and more preferably 9.0 mm or more. On the other hand, it is preferably 15 mm or less, and more preferably 11 mm or less.

In case where the fluid port is formed in the shape of a dialysis liquid side port described in JIS T3250(2012)4.4.4 as aforementioned, although it is preferred that the outer diameter of the portion of the connection part at which is about to reach the fluid port is within a range of 6.0 mm to 9.5 mm as aforementioned, in this case, if the inner diameter of the part to be penetrated is too large, when the connection part is pressed, a deformation of the part to be penetrated being pressed so as to stretch the part to be penetrated having a thin-membrane shape is caused, and it may cause to damage the easiness of opening. Further, if the inner diameter of the part to be penetrated is too small, a space capable of allowing the opened thin membrane to be deformed lacks, and it may cause to damage the easiness of opening. Therefore, the above-described range is preferred.

Since the opened part to be penetrated is deformed so as to be pressed into the interior of the body portion accompanying with the connection part, it is preferred to provide a space for escaping the deformation of the part to be penetrated inside of the body portion in order to achieve an easier opening.

Further, around the part to be penetrated, a lid portion can be formed by forming a synthetic resin frame integrally therewith. Although there is a case where the part to be penetrated cannot be stabilized in shape only by itself because it is frequently formed to be thin, it can be stabilized by being fixed to the synthetic resin frame (for example, a structure as shown in Fig. 9).

In case where the frame in the lid portion and the body portion are formed with a synthetic resin, they can be fixed to each other by ultrasonic welding to form a plug.

Further, as shown in Figs. 8 and 9, by providing a cylindrical cover formed so as to be attached to the fluid port when the plug is attached to the fluid port and forming a projection or a recess capable of engaging the cover and the fluid port with each other, a locking function for preventing an unexpected coming off of the plug can be exhibited from the time of shipping a product up to the time of being used, and it also becomes possible to remove the plug by a simple operation such as pulling out or twisting the plug. Further, it is preferred also in a point that careless touching to the fluid port can be prevented by covering the outer circumference of the fluid port with the cover.

The connection part sealing part is provided inside the lid portion of the plug, and it is a part which liquid-tightly abuts to the connection part at a position where the connection part is pressed, penetrates through the part to be penetrated in the lid portion and is inserted inside the lid portion of the plug. Although the entire surface of the side wall of the plug may be a connection part sealing part, because there is a possibility that the removal of the connection part becomes difficult, as shown in Fig. 7, etc., it is preferred to provide an annular projection, and for example, an embodiment is considered for providing it to the side wall of the body portion or the cover.

It is preferred to form the connection part sealing part in an annular shape because it can be abutted liquid-tightly to the cylindrical part of the connection part. Further, the connection part sealing part is required to enable the liquid flowed in from the connection part to pass through the fluid port and the inside of the module at a state where the connection part sealing part is liquid-tightly abutted to the connection part. At that time, although whether abutting it by attaching the hollow cylindrical connection part sealing part to the outer circumference of the connection part of the fluid treatment device or abutting it by inserting it into the inner circumference can be arbitrarily designed, as aforementioned, it is preferred to abut it to the outer circumference because the size of the connection part sealing part is less restricted.

A good sealing property can be obtained by forming the connection part sealing part with an elastic material having a Shore A hardness of 40 to 90. For example, a synthetic rubber such as a silicone rubber, an EPDM rubber or a nitrile rubber, or an elastomer can be used. A thermoplastic elastomer is preferably used because it can be manufactured at a high accuracy and at a mass scale by using an injection molding.

On the other hand, in a structure where a sealing means (for example, an O-ring, a packing, a gasket) is provided to a connection part of a fluid treatment device and the connection part is abutted to a plug or a fluid port to seal without providing a connection part sealing part to the plug, because there is a possibility that a liquid contact part in the sealing means is unnecessarily polluted by bacteria, hygienic management such as cleaning or exchanging becomes troublesome, and therefore, it does not satisfy the purpose in the present invention.

Further, in the present invention, the connection part sealing part is present inside the lid portion, it is not exposed outside at a state where the connection part is inserted, and therefore, it becomes possible to use it hygienically. Further, because a sealing means is not provided to the connection part, it is not necessary to complicate the shape of the connection part, and the hygienic management thereof such as cleaning or exchanging is extremely easy.

### Examples

Hereinafter, an example with respect to the plug according to the present invention will be described, but the present invention is not limited to this example.

### [Comparative Example 1]

In an ETRF module wherein a bundle of hollow yarn membranes was inserted into a polystyrene resin body case, which had four fluid ports and in which slip-in type fluid ports were provided at two positions in the longitudinal direction and coupler-type fluid ports prepared based on JIS T3250(2011)4.4.4 were provided at two positions in the direction perpendicular to the longitudinal direction, water was filled inside, and EPDM rubber plugs were attached to the four fluid ports, respectively, so as to cover the fluid ports, the EPDM rubber plugs were removed, and connection parts of a dialysis device were attached. Slip-in type connection parts were attached to the fluid ports provided in the longitudinal direction of the module, and coupler-type were attached to the fluid ports provided in the direction perpendicular to the axis of the module.

As a result, the time required for removing the plugs and attaching all the four connection parts was 28 seconds, and the filled liquid leaked by 3 cc.

### [Example 1]

In an ETRF module wherein a bundle of hollow yarn membranes was inserted into a body case, which had four fluid ports and in which the four fluid ports provided in the longitudinal direction of the module and in the direction perpendicular to the longitudinal direction of the module were all coupler-type fluid ports prepared based on JIS T3250(2011)4.4.4, and water was filled inside, the plugs according to the present invention were attached, and connection parts of a dialysis device were attached. Each of the connection parts was formed with a synthetic resin having a pipe shape, and the tip thereof was pressed to a portion near the center of the lid portion and it penetrated therethrough.

The portion near the center of the lid portion of the plug through which the connection part penetrated, the fluid port sealing portion, and the connection part sealing part were formed with a styrene-based thermoplastic elastomer having a Shore A hardness of 70, respectively. The lid portion was formed by two-color integral molding with a synthetic resin frame, and the fluid port sealing portion and the connection part sealing part were formed by two-color integral molding with a synthetic resin cover. The thickness of the portion in the lid portion which was pressed by the connection part, that is, the part to be penetrated, was set at 0.5 mm, and the thickness of the portion around the part to be penetrated was set at 1 mm. A high-density polyethylene was used for the frame, the body portion and the cover, and these were fixed integrally by ultrasonic welding.

The time required for the operation for pressing the connection part to the center of the lid portion and inserting it inside the body with respect to all the four portions was 9 seconds, and the filled liquid did not leak. Further, at the time of the attachment, each of the fluid ports was not exposed outside.

### Industrial Applications of the Invention

The plug according to the present invention can be applied to any medical module in which a plug is detachably attached to a fluid port, and any medical system provided with the module, and in particular, it is suitable for an ETRF module and a medical system provided therewith.

### Explanation of symbols

- 1:: body case
- 2:: header
- 31:: plug
- 311:: opening portion
- 32:: plug
- 4:: hollow yarn membrane
- 51:: fluid port
- 52:: fluid port
- 53:: lure-lock fluid port
- 54:: slip-in fluid port
- 55:: coupler fluid port
- 6:: connection part of dialysis device
- 62:: lure-lock connection part
- 63:: slip-in connection part
- 64:: coupler connection part
- 7:: lid portion
- 71:: part to be penetrated
- 8:: body portion
- 81:: frame
- 82:: body portion
- 9:: connection part sealing part
- 91:: connection part sealing part
- 10:: fluid port sealing portion
- 101:: fluid port sealing portion
- 11:: cover
- 100:: ETRF module
- 500:: fluid port
- 600:: connection part
- 700:: lid portion

## Claims

1. A plug having a body portion, a fluid port sealing portion formed in a hollow cylindrical shape which liquid-tightly abuts a fluid port of a medical module, an opening portion, and a lid portion provided at a side opposite to a side of said opening portion for closing and protecting said fluid port, which is detachably attached to said fluid port of said medical module, **characterized in that**
said lid portion comprises a part to be penetrated through which a cylindrical tubular connection part of a fluid treatment device is capable of penetrating said lid portion, in a state where said plug is attached to said fluid port, when said connection part and said fluid port are connected so as to allow fluid to pass therethrough,
said part to be penetrated includes at least a center part in a radial direction of said lid portion, has a thickness of 0.2 mm to 3.0 mm, and is formed with a soft synthetic resin having a flexural elastic modulus of 100 to 1500 MPa or with an elastic material having a Shore A hardness of 40 to 90, and,
further, said plug comprises a connection part sealing part which liquid-tightly abuts said connection part in a state where said connection part is inserted inside said plug.

2. A plug having a body portion, a fluid port sealing portion formed in a hollow cylindrical shape which liquid-tightly abuts a fluid port of a medical module, an opening portion, and a lid portion provided at a side opposite to a side of said opening portion for closing and protecting said fluid port, which is detachably attached to said fluid port of said medical module, **characterized in that**
said plug comprises a connection part sealing part which liquid-tightly abuts a cylindrical tubular connection part of a fluid treatment device in a state where said connection part is inserted inside said plug, and
said plug is used for a use where said connection part penetrates a part to be penetrated in said lid portion to form a flow path, in a state where said plug is attached to said fluid port, when said fluid port and said connection part are connected so as to allow fluid to pass therethrough without removing said plug from said fluid port.

3. The plug according to claim 2, wherein said part to be penetrated includes at least a center part in a radial direction of said lid portion, has a thickness of 0.2 mm to 3.0 mm, and is formed with a soft synthetic resin having a flexural elastic modulus of 100 to 1500 MPa or with an elastic material having a Shore A hardness of 40 to 90.

4. The plug according to any of claims 1 to 3, wherein said body portion is formed with a synthetic resin in a hollow cylindrical shape, and has said fluid port sealing portion on an inner circumferential surface of said body portion, and said fluid port sealing portion is abutted to an outer circumferential surface of said fluid port when said plug is attached to said fluid port.

5. The plug according to any of claims 1 to 4, wherein said lid portion is molded integrally with a synthetic resin frame, and said frame and said body portion are integrated by ultrasonic welding.

6. A medical module detachably attached with a plug to a fluid port of said medical module, said plug having a body portion, a fluid port sealing portion formed in a hollow cylindrical shape which liquid-tightly abuts said fluid port, an opening portion, and a lid portion provided at a side opposite to a side of said opening portion for closing and protecting said fluid port, **characterized in that**
said lid portion comprises a part to be penetrated through which a cylindrical tubular connection part of a fluid treatment device is capable of penetrating said lid portion, in a state where said plug is attached to said fluid port, when said connection part and said fluid port are connected so as to allow fluid to pass therethrough,
said part to be penetrated includes at least a center part in a radial direction of said lid portion, has a thickness of 0.2 mm to 3.0 mm, and is formed with a soft synthetic resin having a flexural elastic modulus of 100 to 1500 MPa or with an elastic material having a Shore A hardness of 40 to 90, and,
further, said plug comprises a connection part sealing part which liquid-tightly abuts said connection part in a state where said connection part is inserted inside said plug.

7. A medical module detachably attached with a plug to a fluid port of said medical module, said plug having a body portion, a fluid port sealing portion formed in a hollow cylindrical shape which liquid-tightly abuts said fluid port, an opening portion, and a lid portion provided at a side opposite to a side of said opening portion for closing and protecting said fluid port, **characterized in that**
said plug comprises a connection part sealing part which liquid-tightly abuts a cylindrical tubular connection part of a fluid treatment device in a state where said connection part is inserted inside said plug, and
said plug is used for a use where said connection part forms a flow path by penetrating a part to be penetrated in said lid portion, in a state where said plug is attached to said fluid port, when said fluid port and said connection part are connected so as to allow fluid to pass therethrough without removing said plug from said fluid port.

8. The medical module according to claim 7, wherein said part to be penetrated includes at least a center part in a radial direction of said lid portion, has a thickness of 0.2 mm to 3.0 mm, and is formed with a soft synthetic resin having a flexural elastic modulus of 100 to 1500 MPa or with an elastic material having a Shore A hardness of 40 to 90.

9. The medical module according to any of claims 6 to 8, wherein said medical module is an endotoxin retentive filter module.

10. A medical system comprising (a) a medical module detachably attached with a plug to a fluid port of said medical module, said plug having a body portion, a fluid port sealing portion formed in a hollow cylindrical shape which liquid-tightly abuts said fluid port, an opening portion, and a lid portion provided at a side opposite to a side of said opening portion for closing and protecting said fluid port, and (b) a fluid treatment device, **characterized in that**
a cylindrical tubular connection part of said fluid treatment device is penetrated through a part to be penetrated including at least a center part in a radial direction of said lid portion, and inserted inside said plug, and
said inserted connection part is liquid-tightly abutted to a connection part sealing part, which is provided inside said plug, to open a flow path.
